# EUROPÄISCHE PATENTSCHRIFT

(11) **EP 3 001 972 B1**
(45) Veröffentlichungstag und Bekanntmachung des Hinweises auf die Patenterteilung: **27.09.2017**
(21) Anmeldenummer: 15003020.3
(22) Anmeldetag: 01.08.2006
(51) Int. Cl.: A61B 34/00, A61F 9/007, B25J 13/02, G01L 5/22, A61B 17/00

(54) **SERVOGESTÜTZTER MEDIZINISCHER APPLIKATOR**
SERVO-ASSISTED MEDICAL APPLICATOR
APPLICATEUR MEDICAL ASSERVI

(43) Veröffentlichungstag der Anmeldung: 06.04.2016
(62) Teilanmeldung aus: 06015985.2
(73) Patentinhaber: WaveLight GmbH, 91058 Erlangen (DE)
(72) Erfinder: Kittelmann, Olaf, 14163 Berlin (DE); Robl, Gerhard, 90547 Stein (DE); Vogler, Klaus, 90542 Eckental (DE)
(74) Vertreter: Frenkel, Matthias Alexander

(56) Entgegenhaltungen:
- US-A1- 2004 128 026
- US-A1- 2005 222 587
- US-B1- 6 665 554

## Beschreibung

Die Erfindung betrifft das motorunterstützte Positionieren einer medizinischen Vorrichtung an einem Körperteil oder einem Organ eines Patienten.

Für bestimmte medizinische Behandlungen muss eine medizinische Vorrichtung an einem Körperteil oder einem Organ eines Patienten präzise und vorsichtig positioniert werden. Ein Beispiel einer derartigen medizinischen Behandlung ist die refraktive Augenchirurgie, bei der mittels eines Laserstrahls aus der Hornhaut Gewebe entfernt wird, um eine Fehlsichtigkeit des Patienten zu korrigieren.

An einem Körperteil oder einem Organ eines Patienten zu positionierende medizinische Vorrichtungen werden typischerweise mittels gelenkiger Stützvorrichtungen in der gewünschten Position gehalten. Diese gelenkigen Stützvorrichtungen müssen in jedem Gelenk ein erhebliches Maß an Reibung aufweisen, damit die medizinische Vorrichtung in ihrer Position gehalten wird und sich nicht selbständig bewegt. Das Behandlungspersonal muss zum Positionieren der medizinischen Vorrichtung folglich die Reibung in den Gelenken und die aus der Masse der medizinischen Vorrichtung resultierende Trägheit überwinden, um die medizinische Vorrichtung zu positionieren. Insbesondere gestaltet sich eine geringfügige Änderung der Position der medizinischen Vorrichtung schwierig, da zum Überwinden der Reibung in den einzelnen Gelenken und der Massenträgheit der medizinischen Vorrichtung ein größeres Maß an Muskelkraft erforderlich ist, was häufig zu einer ruckartigen Bewegung führt, so dass die medizinische Vorrichtung nicht die gewünschte Position einnimmt und der Patient einen Schmerz empfindet und/oder schlimmstenfalls ein empfindliches Organ des Patienten verletzt wird.

Es wurde vorgeschlagen, eine medizinische Vorrichtung mittels eines Joysticks an einem Behandlungsort zu positionieren, wobei die Stützvorrichtung mittels Schrittmotoren positioniert wird. Jedoch hat das Behandlungspersonal bei einem derartigen Positionierungsverfahren keine direkte Rückkopplung zur medizinischen Vorrichtung und kann nicht feststellen, mit welcher Kraft bzw. mit welchem Impuls die medizinische Vorrichtung auf einem Körperteil bzw. einem Organ des Patienten auftrifft, wodurch ein Schmerz empfunden werden kann und schlimmstenfalls eine Verletzung entstehen kann. Der Joystick gestattet zwar eine präzise Positionierung, verhindert aber einen direkten Kontakt eines Verwenders mit der medizinischen Vorrichtung.

Der Verwender der medizinischen Vorrichtung kann somit bei der Betätigung des Joysticks nicht beurteilen, ob die medizinische Vorrichtung an einem Körperteil eines Patienten angestoßen ist, was zu einem Schmerz und/oder einer Verletzung führen kann. Ferner wurde vorgeschlagen, das genaue Positionieren der medizinischen Vorrichtung mit Hilfe einer Druckmessung und einer entsprechenden Regelung zu verbessern. Diese Regelung weist jedoch eine erhebliche Trägheit wegen der bewegten Massen auf, und zudem kann diese Regelung erst einsetzen, wenn das Körperteil bzw. das Organ eines Patienten berührt wird, was bei besonders empfindlichen Organen zu spät sein kann und trotzdem eine unter Umständen irreversible Verletzung des empfindlichen Organs verursachen kann. Diverse Steuerungen von medizinischen System sind beispielsweise aus den Druckschriften US-A-2004/128026, US-A-6665554, und US-A-2005/222587 bekannt. Es ist somit eine Aufgabe der Erfindung, eine Vorrichtung und ein Verfahren zum exakten Positionieren einer medizinischen Vorrichtung an einem Körperteil oder einem Organ eines Patienten zu schaffen.

Die Aufgabe wird durch ein medizinisches System gelöst, das eine medizinische Vorrichtung, eine Führungseinrichtung und eine Krafterfassungseinrichtung umfasst, die mit der Führungseinrichtung und der medizinischen Vorrichtung mechanisch gekoppelt ist.

Bei einem Verfahren zum Positionieren der medizinischen Vorrichtung wird eine Kraft erfasst, die auf die mit der medizinischen Vorrichtung mechanisch gekoppelte Führungseinrichtung wirkt, und eine kinematische Größe der medizinischen Vorrichtung wird in Abhängigkeit von der erfassten Kraft mit einer Antriebseinrichtung geändert.

Die medizinische Vorrichtung kann eine ophthalmologische Vorrichtung sein, beispielsweise eine Laservorrichtung für die refraktive Augenchirurgie, ein mechanisches Mikrokeratom zum Erzeugen eines Schnittes in der Hornhaut eines Auges eines Patienten, ein Augensaugring, ein auf einem Femtosekundenlaser basierendes optisches Mikrokeratom, das mittels Lichtimpulse einen Schnitt in der Hornhaut eines Patienten erzeugt.

Die Krafterfassungseinrichtung kann eine Kraft eindimensional, zweidimensional und/oder dreidimensional messen. Die Krafterfassungseinrichtung kann einen oder eine Mehrzahl von Druck- und/oder Kraftsensoren umfassen. Es können beispielsweise sogenannte Dehnungsmessstreifen verwendet werden.

Der Ausdruck Führungseinrichtung umfasst jegliche Einrichtung, die dazu geeignet ist, dass ein Verwender damit durch Berührung der Führungseinrichtung und/oder durch Aufbringen einer Kraft auf die Führungseinrichtung die medizinische Vorrichtung zu einem Behandlungsort führen kann. Die Führungseinrichtung kann als Greifeinrichtung, beispielsweise als Handgriff, ausgebildet sein. Es ist auch möglich, die Krafterfassungseinrichtung flächig an zumindest einem Bereich der Außenfläche der medizinischen Vorrichtung anzuordnen, wobei die Außenfläche der Krafterfassungseinrichtung hierbei als Führungseinrichtung wirkt. Eine derartige Krafterfassungseinrichtung kann beispielsweise eine Matrix von Drucksensoren bzw. Kraftsensoren sein.

Es versteht sich, dass jeder starre Körper eine gewisse Elastizität aufweist. Sogar ein starrer Körper verformt sich geringfügig, falls eine Kraft auf ihn wirkt. Eine derartige Verformung kann beispielsweise durch Dehnungsmessstreifen ermittelt werden. Somit kann beispielsweise eine von einem Verwender aufgebrachte Kraft auf eine Führungseinrichtung durch die geringfügige Verformung der Führungseinrichtung bestimmt werden. Ferner kann eine (geringfügige) elastische Kopplung zwischen der Führungseinrichtung und der medizinischen Vorrichtung vorgesehen werden, wodurch sich eine genauere Bestimmung der von einem Verwender auf die Führungseinrichtung aufgebrachten Kraft ergeben kann.

Die medizinische Vorrichtung kann mit einer Antriebseinrichtung gekoppelt sein, wobei die Antriebseinrichtung eine kinematische Größe der medizinischen Vorrichtung in Abhängigkeit von einer von der Krafterfassungseinrichtung erfassten Kraft ändert.

Die Antriebseinrichtung bewegt die medizinische Vorrichtung in die Richtung der erfassten Kraft, solange ein Verwender der medizinischen Vorrichtung eine Kraft auf die Führungseinrichtung aufbringt. Dadurch wird der Verwender der medizinischen Vorrichtung entlastet, da er nur noch eine geringere Muskelkraft aufbringen muss und die Muskelkraft nicht die Reibung in den Gelenken und/oder Achsen einer Stützvorrichtung und die Massenträgheit der medizinischen Vorrichtung überwinden muss. Dadurch entsteht auch bei einer Feinpositionierung keine ruckartige Bewegung, die zu einer Verletzung eines empfindlichen Organs eines Patienten führen kann. Bei dem erfindungsgemäßen medizinischen System ist eine leichtgängige Bewegung mit der erforderlichen Präzision möglich und der Verwender des medizinischen Systems hat einen direkten Kontakt zur medizinischen Vorrichtung, wodurch der Verwender leicht beurteilen kann, ob die medizinische Vorrichtung ein Körperteil eines Patienten berührt. Dadurch wird ein Schmerz beim Patienten oder eine Verletzung eines Körperteils des Patienten vermieden.

Die Änderung der kinematischen Größe kann zumindest teilweise eine kontinuierliche Funktion der Kraft sein. Die kinematische Größe kann eine Beschleunigung, eine Geschwindigkeit und/oder eine Position sein. Die Änderung der kinematischen Größe kann durch eine Steuerung oder durch eine (zurückgekoppelte) Regelung bewirkt werden. Beispielsweise kann in Abhängigkeit von der erfassten Kraft der Sollwert der Beschleunigung, der Geschwindigkeit oder der Position geändert werden. Dem Fachmann sind die dafür notwendigen Steuerungsalgorithmen und Regelungsalgorithmen bekannt. Bei einer besonders einfachen Ausführungsform kann lediglich die Richtung der Kraft bestimmt werden und als Reaktion auf die erfasste Kraftrichtung der Sollwert der Position um einen konstanten Betrag geändert werden. Die Antriebseinrichtung kann einen Schrittmotor umfassen, wobei sich aus der Frequenz der Schrittimpulse die Beschleunigung, die Geschwindigkeit und folglich die Positionsänderung ergibt. Die Änderung der kinematischen Größe kann innerhalb des bei der jeweiligen Steuerungseinrichtung bzw. Regelungseinrichtung möglichen Wertebereiches eine kontinuierliche Funktion der Kraft sein. Die Änderung der kinematischen Größe kann beispielsweise innerhalb des Wertebereiches für einen Sollwert eine lineare Funktion, eine Polynomfunktion oder eine exponentielle Funktion sein. Es versteht sich, dass der Begriff kontinuierliche Funktion auch eine digitale Darstellung, beispielsweise eine zeitdiskrete und/oder amplitudendiskrete Darstellung, der Funktionswerte umfasst.

Die Änderung der kinematischen Größe kann auch eine stufenartige Funktion der Kraft sein.

Die Antriebseinrichtung muss nicht Teil der medizinischen Vorrichtung, sondern nur Teil des medizinischen Systems sein. Die medizinische Vorrichtung kann an einer Stützvorrichtung mit zumindest einem Gelenk angeordnet sein, wobei die Antriebseinrichtung das zumindest eine Gelenk der Stützvorrichtung bewegt. Die medizinische Vorrichtung kann auch an einem XY-Portal mit einer beweglichen X-Achse, einer beweglichen Y-Achse und einer ausfahrbaren Z-Achse angeordnet sein, wobei die Antriebseinrichtung eine kinematische Größe der einzelnen Achsen ändert. Es können auch rotatorische Achsen vorgesehen sein. Achse im Zusammenhang dieser Erfindung bedeutet Freiheitsgrad der Stützvorrichtung.

Die mit der medizinischen Vorrichtung gekoppelte Antriebseinrichtung kann derart ausgebildet sein, dass sie die medizinische Vorrichtung verzögert bzw. abbremst, falls keine Kraft erfasst wird. Dadurch wird die medizinische Vorrichtung sicher abgebremst, so dass keine Verletzung eines Organs oder eines Körperteiles des Patienten auftritt und/oder der Patient keinen Schmerz verspürt.

Die mit der medizinischen Vorrichtung gekoppelte Antriebseinrichtung kann eine Lageregelung durchführen, falls keine auf die Krafterfassungseinrichtung wirkende Kraft und keine Bewegung der medizinischen Vorrichtung erfasst wird. Durch die Lageregelung wird die medizinische Vorrichtung sicher in der gewünschten Position gehalten, so dass keine Reibung in dem zumindest einen Freiheitsgrad der Stützvorrichtung erforderlich ist. Die Lageregelung schafft somit aus Sicht des Verwenders eine künstliche Reibung.

Die Führungseinrichtung kann an der medizinischen Vorrichtung angeordnet sein. Da die Führungseinrichtung an der medizinischen Vorrichtung angeordnet ist, kann die medizinische Vorrichtung besonders genau positioniert werden.

Das medizinische System kann dadurch hergestellt werden, dass eine medizinische Vorrichtung über einen Kraftsensor mit einer Führungseinrichtung mechanisch gekoppelt wird. Die Krafterfassungseinrichtung und die Führungseinrichtung können wie zuvor beschrieben ausgebildet sein. Die medizinische Vorrichtung kann mit der Stützvorrichtung und der Antriebseinrichtung gekoppelt sein.

Wie zuvor erwähnt wurde, kann das medizinische System die Stützvorrichtung mit zumindest einem Freiheitsgrad, an dem die medizinische Vorrichtung angeordnet ist, umfassen, wobei die Antriebseinrichtung eine Kraft auf die Stützvorrichtung in Richtung des zumindest einen Freiheitsgrades ausübt. Bei einem derartigen medizinischen System ist die Führungseinrichtung an der medizinischen Vorrichtung angeordnet. Ein weiteres medizinisches System umfasst die Stützvorrichtung mit zumindest einem Freiheitsgrad, an dem die medizinische Vorrichtung angeordnet ist, wobei die Antriebseinrichtung eine Kraft auf die Stützvorrichtung in Richtung des zumindest einen Freiheitsgrades ausübt und wobei bei diesem medizinischen System die Führungseinrichtung an der Stützvorrichtung angeordnet ist. Diese Stützvorrichtung kann bei einer Mehrzahl von medizinischen Vorrichtungen unterschiedlichen Typs verwendet werden.

Die Erfindung wird jetzt unter Bezugnahme auf die begleitenden Zeichnungen detaillierter beschrieben, wobei
Fig. 1 eine Ausführungsform, bei der eine Führungseinrichtung an der medizinischen Vorrichtung angeordnet ist, und
Fig. 2 Bei der Erfindung ist die Führungseinrichtung an einer Stützvorrichtung mit drei Freiheitsgraden angeordnet ist.

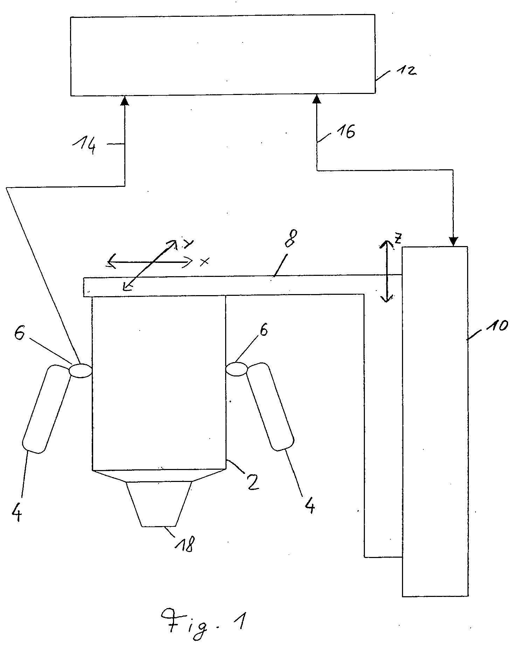

Fig. 1 zeigt eine medizinische Vorrichtung 2 mit einer Behandlungsöffnung 18. Die medizinische Vorrichtung 2 kann eine ophthalmologische Einrichtung sein, beispielsweise eine Laservorrichtung für die refraktive Augenchirurgie, ein mechanisches Mikrokeratom zum Erzeugen eines Schnittes in der Hornhaut eines Auges eines Patienten oder ein auf einem Femtosekundenlaser basierendes optisches Mikrokeratom, das mittels Lichtimpulse einen Schnitt in der Hornhaut eines Patienten erzeugt. In der Behandlungsöffnung 18 kann sich beispielsweise die Schneidklinge eines mechanischen Mikrokeratoms befinden oder an der Behandlungsöffnung 18 kann ein Lichtstrahl, beispielsweise ein Laserstrahl, austreten.

Die medizinische Vorrichtung ist an einer Stützvorrichtung 8 mit drei Freiheitsgraden x, y, z angeordnet. Eine Antriebseinrichtung 10 kann eine Kraft in Richtung jedes der drei Freiheitsgrade der Stützvorrichtung 8 erzeugen. Eine Führungseinrichtung 4 ist über eine Krafterfassungseinrichtung 6 mit der medizinischen Vorrichtung 2 gekoppelt. Bei dieser Ausführungsform eines medizinischen Systems ist die Führungseinrichtung 4 und die Krafterfassungseinrichtung 6 an der medizinischen Vorrichtung 2 angeordnet. Diese Ausführungsform umfasst ferner eine Mehrzahl von Führungseinrichtungen 4 und Krafterfassungseinrichtungen 6, was aber nicht notwendigerweise der Fall sein muss. Die Führungseinrichtung 4 ist als Handgriff ausgebildet, es sind jedoch, wie zuvor erwähnt, andere Ausgestaltungen der Führungseinrichtung 4 möglich, beispielsweise ein Muldengriff oder ein Bügelgriff. Die Führungseinrichtung 4 kann auch integral mit der Krafterfassungseinrichtung 6 ausgebildet werden, indem beispielsweise die Krafterfassungseinrichtung flächig ausgebildet ist und zumindest an einem Bereich der Außenfläche der medizinischen Vorrichtung 2 angeordnet ist. Eine derartige Krafterfassungseinrichtung kann beispielsweise aus einer Drucksensormatrix oder Kraftsensormatrix aufgebaut sein.

Bei dieser Ausführungsform erfasst jede Krafterfassungseinrichtung 6 die Kraft in allen drei Raumrichtungen x, y, z. Es kann auch eine Mehrzahl von Krafterfassungseinrichtungen 6 vorgesehen werden, die eine Kraft eindimensional oder zweidimensional erfassen. Die Krafterfassungseinrichtung könnte auch eine Kraft bzw. ein Drehmoment um eine rotatorische Achse erfassen. Eine Verarbeitungseinrichtung 12 erhält die von der zumindest einen Krafterfassungseinrichtung 6 ausgegebenen Signale, die über eine Leitung 14 an die Verarbeitungseinrichtung 12 übertragen werden. Die Verarbeitungseinrichtung 12 kann als Regelungseinrichtung oder als Steuerungseinrichtung ausgebildet sein. Die Verarbeitungseinrichtung 12 überträgt über eine Leitung 16 Steuersignale an die Antriebseinrichtung 10.

Versucht ein Verwender der medizinischen Vorrichtung 2 diese durch Anlegen einer Kraft auf zumindest eine der Führungseinrichtungen 4 zu verschieben, erfasst zumindest eine Krafterfassungseinrichtung 6 diese Kraft und überträgt ein entsprechendes Signal über die Leitung 14 an die Verarbeitungseinrichtung 12. Die Verarbeitungseinrichtung 12 ändert zumindest einen Sollwert einer kinematischen Größe der Antriebseinrichtung 10. Beispielsweise kann der Sollwert für eine Beschleunigung, eine Geschwindigkeit oder eine Position zumindest einer Achse der Stützvorrichtung 8 mit drei Freiheitsgraden geändert werden. Es versteht sich, dass die Antriebseinrichtung 10 und/oder die Stützvorrichtung 8 eine Mehrzahl von Sensoren umfassen können, die den Istwert der Beschleunigung, der Geschwindigkeit und/oder der Position einer Achse erfassen. Die erfassten Istwerte können über die Leitung 16 an die Verarbeitungseinrichtung 12 übertragen werden. Dadurch kann die Verarbeitungseinrichtung 12 eine kinematische Größe der medizinischen Vorrichtung 2 in Richtung des zumindest einen Freiheitsgrades der Stützvorrichtung 8 regeln. Es versteht sich jedoch, dass für die Zwecke dieser Erfindung eine Regelung nicht notwendigerweise erforderlich ist, sondern eine Steuerung zumindest einer kinematischen Größe der medizinischen Vorrichtung 2 in Richtung des zumindest einen Freiheitsgrades der Stützvorrichtung 8 ausreichend ist. Die Antriebseinrichtung 10 kann zumindest einen elektrischen und/oder pneumatischen Antrieb umfassen.

Die medizinische Vorrichtung 2 wird folglich unterstützt durch die Kraft der Antriebseinrichtung 10 in die vom Verwender gewünschte Richtung bewegt, so dass der Verwender weniger Kraft aufwenden muss, um die medizinische Vorrichtung 2 zu bewegen, keine ruckartige Bewegung entsteht, da keine oder nur eine verringerte Massenträgheit der medizinischen Vorrichtung 2 und der Stützvorrichtung 8 überwunden werden muss und keine oder nur eine verringerte Reibung in der Stützvorrichtung 8 überwunden werden muss, und die Behandlungsöffnung 18 kann präzise an dem zu behandelnden Organ oder Körperteil, beispielsweise dem Auge, eines Patienten positioniert werden. Da sich die Kraft verringert, mit der der Verwender, bei dem es sich um medizinisches Hilfspersonal handeln kann, die medizinische Vorrichtung 2 positioniert, kann der Verwender die medizinische Vorrichtung 2 besonders genau positionieren. Damit ist die Verletzung eines zu behandelnden Organs oder Körperteiles weitgehend ausgeschlossen.

Übt der Verwender keine Kraft auf die Führungseinrichtungen 4 aus, gibt die Verarbeitungseinrichtung 12 ein Signal an die Antriebseinrichtung 10 aus, damit diese die Bewegung der medizinischen Vorrichtung 2 und der Stützvorrichtung 8 bremst (verzögert) und/oder zum Stillstand bringt. Bewegt sich die medizinische Vorrichtung 2 nicht und wird keine Kraft auf die Führungseinrichtungen 4 ausgeübt, führt die Verarbeitungseinrichtung 12 eine Lageregelung durch, die die medizinische Vorrichtung 2 in ihrer Position hält. Sobald der Verwender eine ausreichend hohe Kraft auf zumindest eine der Führungseinrichtungen 4 aufbringt, wird die Lageregelung abgeschaltet und die Verarbeitungseinrichtung 12 führt die zuvor genannten Schritte zum Verändern der Position der medizinischen Vorrichtung 2 durch.

Aus Sicherheitsgründen kann bei einem Stromausfall während der Lageregelung eine mechanische Bremse (nicht gezeigt) aktiviert werden, die die medizinische Vorrichtung 2 in ihrer Position hält. Ferner kann eine mechanische Bremse betätigt werden, sobald der Verwender keine Kraft auf die Führungseinrichtung aufbringt. Diese mechanische Bremse kann auch betätigt werden, solange der Verwender keine Kraft auf die Führungseinrichtung aufbringt und sich die Führungseinrichtung nicht bewegt.

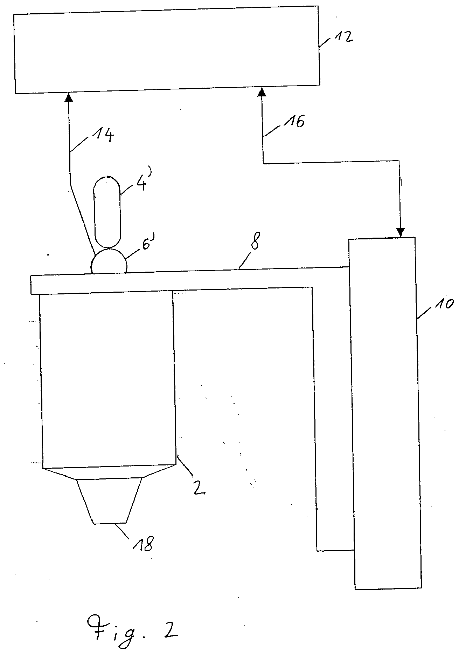

Fig. 2 zeigt eine zweite Ausführungsform eines medizinischen Systems, wobei gleiche oder ähnliche Elemente mit den gleichen Bezugszeichen wie in Fig. 1 gekennzeichnet sind. Bei der zweiten Ausführungsform ist eine Führungseinrichtung 4' über eine Krafterfassungseinrichtung 6' und zumindest einen Teil der Stützvorrichtung 8 mit der medizinischen Vorrichtung 2 gekoppelt. Die Funktionsweise der zweiten Ausführungsform gemäß Fig. 2 entspricht der Funktionsweise der ersten Ausführungsform 1. Die Erfindung umfasst auch die zweite Ausführungsform gemäß Fig. 2.

Es ist ein Vorteil der vorliegenden Erfindung, dass eine medizinische Vorrichtung 2 präziser an einem Organ oder einem Körperteil eines Patienten positioniert werden kann, ohne dem Patienten einen Schmerz zuzufügen und/oder das Organ oder das Körperteil zu verletzen. Der Verwender, bei dem es sich um medizinisches Hilfspersonal handeln kann, muss beim Positionieren der medizinischen Vorrichtung 2 nicht die Massenträgheit der medizinischen Vorrichtung 2 und der Stützvorrichtung 8 sowie die Reibung in der Stützvorrichtung 8 überwinden, wodurch nur ein geringerer Kraftaufwand zum Positionieren der medizinischen Vorrichtung erforderlich ist und die medizinische Vorrichtung 2 ruckfrei am Behandlungsort positioniert werden kann. In beiden Ausführungsformen ist eine Stützvorrichtung 8 mit drei linearen Freiheitsgraden 8 gezeigt. Es versteht sich, dass die Stützvorrichtung 8 lineare sowie rotatorische Freiheitsgrade oder nur rotatorische Freiheitsgrade in der Form von Gelenken aufweisen kann.

## Patentansprüche

1. Verfahren zum Positionieren einer medizinischen Vorrichtung (2) mit den Schritten:
Erfassen einer Kraft, die auf eine mit der medizinischen Vorrichtung (2) mechanisch gekoppelte Führungseinrichtung (4') wirkt, mit einer Krafterfassungseinrichtung (6'), die zwischen der Führungseinrichtung (4') und der medizinischen Vorrichtung (2) angeordnet ist, und Ändern einer kinematischen Größe der medizinischen Vorrichtung (2) durch eine Antriebseinrichtung in Abhängigkeit von der erfassten Kraft, wobei eine Stützvorrichtung (8) mit zumindest einem Freiheitsgrad, an der medizinischen Vorrichtung (2) angeordnet ist, wobei die Antriebseinrichtung (10) eine Kraft auf die Stützvorrichtung (8) in Richtung des zumindest einen Freiheitsgrades ausübt, wobei die Führungseinrichtung (4') über die Krafterfassungseinrichtung (6') und zumindest einen Teil der Stützvorrichtung an die medizinische Vorrichtung gekoppelt ist, **gekennzeichnet durch** die Schritte: Bremsen der medizinischen Vorrichtung (2) und der Stützvorrichtung (8) durch die Antriebseinrichtung falls keine Kraft erfasst wird, wobei
eine Verarbeitungseinrichtung (12) ein Signal zur Betätigung an eine mechanischen Bremse ausgibt, die die medizinische Vorrichtung (2) in ihrer Position hält, falls keine auf die Führungseinrichtung wirkende Kraft ermittelt wird, und ermittelt wird, dass sich die medizinische Vorrichtung (2) nicht bewegt.

2. Verfahren nach Anspruch 1, wobei die Änderung der kinematischen Größe zumindest teilweise eine Funktion der Kraft ist.

3. Verfahren nach Anspruch 1 oder 2, wobei die kinematische Größe eine Beschleunigung, eine Geschwindigkeit und/oder eine Position ist.

4. Verfahren nach einem der vorstehenden Ansprüche, weiterhin umfassend den Schritt des Regelns der Lage der medizinischen Vorrichtung, falls keine auf die Krafterfassungseinrichtung (6') wirkende Kraft und keine Bewegung der medizinischen Vorrichtung erfasst wird.

5. Medizinisches System, umfassend
eine medizinische Vorrichtung (2),
eine Führungseinrichtung (4')
eine Krafterfassungseinrichtung (6'), die mit der Führungseinrichtung (4') und der medizinischen Vorrichtung (2) mechanisch gekoppelt ist,
eine Verarbeitungseinrichtung (12), die eingerichtet ist, um ein Signal zur Betätigung einer mechanischen Bremse auszugeben,
eine Antriebseinrichtung (10), die eingerichtet ist, eine kinematische Größe der medizinischen Vorrichtung (2) in Abhängigkeit von einer von der Krafterfassungseinrichtung (6') erfassten Kraft zu ändern,
eine Stützvorrichtung (8) mit zumindest einem Freiheitsgrad, an der die medizinische Vorrichtung (2) angeordnet ist, wobei die Antriebseinrichtung (10) eingerichtet ist, um eine Kraft auf die Stützvorrichtung (8) in Richtung des zumindest einen Freiheitsgrades auszuüben, wobei die Führungseinrichtung (4') über die Krafterfassungseinrichtung (6') über zumindest einen Teil der Stützvorrichtung an die medizinische Vorrichtung gekoppelt ist, und eine Verarbeitungseinrichtung, **dadurch gekennzeichnet, dass** die mit der medizinischen Vorrichtung gekoppelte Antriebseinrichtung (10) derart ausgebildet ist, dass sie die medizinische Vorrichtung (2) bremst, falls keine Kraft erfasst wird, und
die medizinische Vorrichtung eine mechanische Bremse umfasst, die eingerichtet ist, um die medizinische Vorrichtung (2) in ihrer Position zu halten, falls sie durch die Verarbeitungseinrichtung betätigt wird, wobei die Bremse betätigt wird, falls ermittelt wird, dass keine Kraft auf die Führungseinrichtung (4') wirkt und sich die medizinische Vorrichtung nicht bewegt.

6. Medizinisches System nach Anspruch 5,
wobei die mit der medizinischen Vorrichtung (2) gekoppelte Antriebseinrichtung (10) derart ausgebildet ist, dass sie in Abhängigkeit von der erfassten Kraft die Beschleunigung, die Geschwindigkeit und/oder die Position der medizinischen Vorrichtung (2) ändert.

7. Medizinisches System nach Anspruch 5 oder 6,
wobei die Antriebseinrichtung (10) derart ausgebildet ist, dass sie die kinematische Größe zumindest teilweise als Funktion der von der Krafterfassungseinrichtung (6') erfassten Kraft ändert.

8. Medizinisches System nach einem der Ansprüche 5 bis 7,
wobei die mit der medizinischen Vorrichtung (2) gekoppelte Antriebseinrichtung (10) eine Lageregelung durchführt, falls keine auf die Krafterfassungseinrichtung (6') wirkende Kraft und keine Bewegung der medizinischen Vorrichtung (2) erfasst wird.

9. Medizinisches System nach einem der Ansprüche 5 bis 8,
wobei die Führungseinrichtung (6) an der medizinischen Vorrichtung (2) angeordnet ist.

## Claims

1. Method for positioning a medical device (2), with the steps of:
detecting a force, which acts on a guide unit (4') mechanically coupled to the medical device (2), using a force detection unit (6') arranged between the guide unit (4') and the medical device (2), and
changing a kinematic variable of the medical device (2) by means of a drive unit in accordance with the detected force,
wherein an assistance device (8) with at least one degree of freedom is arranged on the medical device (2), wherein the drive unit (10) exerts a force on the assistance device (8) in the direction of the at least one degree of freedom, wherein the guide unit (4') is coupled to the medical device via the force detection unit (6') and at least part of the assistance device,
**characterized by** the steps of:
braking the medical device (2) and the assistance device (8) by means of the drive unit if no force is detected, wherein a processor (12) outputs an actuation signal to a mechanical brake which holds the medical device (2) in its position if no force acting on the guide unit is determined and if it is determined that the medical device (2) is not moving.

2. Method according to Claim 1, wherein the change of the kinematic variable is at least partially a function of the force.

3. Method according to Claim 1 or 2, wherein the kinematic variable is an acceleration, a speed and/or a position.

4. Method according to one of the preceding claims, moreover comprising the step of controlling the position of the medical device if no force acting on the force detection unit (6') is detected and no movement of the medical device is detected.

5. Medical system comprising
a medical device (2),
a guide unit (4'),
a force detection unit (6') which is mechanically coupled to the guide unit (4') and to the medical device (2),
a processor (12) which is configured to output a signal for actuation of a mechanical brake,
a drive unit (10) which is configured to change a kinematic variable of the medical device (2) in accordance with a force detected by the force detection unit (6'),
an assistance device (8) which has at least one degree of freedom and on which the medical device (2) is arranged, wherein the drive unit (10) is configured to exert a force on the assistance device (8) in the direction of the at least one degree of freedom, wherein the guide unit (4') is coupled to the medical device via the force detection unit (6') and at least part of the assistance device, and a processor, **characterized in that** the drive unit (10) coupled to the medical device is designed in such a way that it brakes the medical device (2) if no force is detected, and the medical device comprises a mechanical brake which is configured to hold the medical device (2) in its position if it is actuated by the processor, wherein the brake is actuated if it is determined that no force is acting on the guide unit (4') and the medical device is not moving.

6. Medical system according to Claim 5, wherein the drive unit (10) coupled to the medical device (2) is designed in such a way that it changes the acceleration, the speed and/or the position of the medical device (2) in accordance with the detected force.

7. Medical system according to Claim 5 or 6, wherein the drive unit (10) is designed in such a way that it changes the kinematic variable at least partially as a function of the force detected by the force detection device (6').

8. Medical system according to one of Claims 5 to 7, wherein the drive unit (10) coupled to the medical device (2) performs a position control if no force acting on the force detection device (6') is detected and no movement of the medical device (2) is detected.

9. Medical system according to one of Claims 5 to 8, wherein the guide unit (6) is arranged on the medical device (2).

## Revendications

1. Procédé destiné au positionnement d'un dispositif médical (2), lequel procédé comprend les phases suivantes :
la détection d'une force, laquelle agit sur un mécanisme de guidage (4'), qui est couplé mécaniquement au dispositif médical (2), au moyen d'un mécanisme de détection de la force (6') qui est disposé entre le mécanisme de guidage (4') et le dispositif médical (2) ; et
la modification d'une grandeur cinématique du dispositif médical (2) par l'intermédiaire d'un mécanisme d'entraînement en fonction de la force détectée, selon lequel un dispositif de soutien (8) comprenant tout au moins un degré de liberté est disposé au niveau du dispositif médical (2), selon lequel le mécanisme d'entraînement (10) exerce une force sur le dispositif de soutien (8) en direction du tout au moins un degré de liberté, selon lequel le mécanisme de guidage (4') est couplé au dispositif médical par l'intermédiaire du mécanisme de détection de la force (6') et de tout au moins une partie du dispositif de soutien,
**caractérisé par** les phases suivantes : le freinage du dispositif médical (2) et du dispositif de soutien (8) par l'intermédiaire du mécanisme d'entraînement quand aucune force n'est détectée, selon lequel un mécanisme de traitement (12) émet un signal destiné à la réalisation d'une manoeuvre au niveau d'un frein mécanique, lequel maintient le dispositif médical (2) dans sa position quand aucune force agissant sur le mécanisme de guidage n'est détectée et quand aucun mouvement du dispositif médical (2) n'est détecté.

2. Procédé selon la revendication 1,
selon lequel la modification de la grandeur cinématique est, tout au moins en partie, une fonction de la force.

3. Procédé selon la revendication 1 ou 2,
selon lequel la grandeur cinématique est une accélération, une vitesse et/ou une position.

4. Procédé selon l'une des revendications précédentes, comprenant en outre la phase du réglage du positionnement du dispositif médical quand aucune force agissant sur le mécanisme de détection de la force (6') n'est détectée et quand aucun mouvement du dispositif médical n'est détecté.

5. Système médical comprenant :
- un dispositif médical (2) ;
- un mécanisme de guidage (4') ;
- un mécanisme de détection de la force (6') qui est couplé mécaniquement avec le mécanisme de guidage (4') et le dispositif médical (2) ;
- un mécanisme de traitement (12) qui est réglé de manière à émettre un signal en vue de l'actionnement d'un frein mécanique ;
- un mécanisme d'entraînement (10) qui est réglé de manière à modifier une grandeur cinématique du dispositif médical (2) en fonction d'une force détectée par le mécanisme de détection de la force (6') ;
- un dispositif de soutien (8) avec tout au moins un degré de liberté, au niveau duquel dispositif de soutien le dispositif médical (2) est disposé, selon lequel le mécanisme d'entraînement (10) est réglé de manière à exercer une force sur le dispositif de soutien (8) dans la direction du tout au moins un degré de liberté, selon lequel le mécanisme de guidage (4') est couplé au dispositif médical par l'intermédiaire du mécanisme de détection de la force (6') et de tout au moins une partie du dispositif de soutien ; et
- un mécanisme de traitement,
**caractérisé en ce que**
le mécanisme d'entraînement (10) couplé avec le dispositif médical est conçu de telle sorte qu'il freine le dispositif médical (2) quand aucune force n'est détectée ; et
**caractérisé en ce que**
le dispositif médical comprend un frein mécanique qui est réglé de manière à maintenir le dispositif médical (2) dans sa position quand il est actionné par l'intermédiaire du mécanisme de traitement, selon lequel le frein est actionné quand il s'avère par la détection qu'aucune force n'agit sur le mécanisme de guidage (4') et quand le dispositif médical n'est pas en mouvement.

6. Système médical selon la revendication 5,
selon lequel le mécanisme d'entraînement (10) couplé avec le dispositif médical (2) est conçu de telle sorte qu'il modifie l'accélération, la vitesse et/ou la position du dispositif médical (2) en fonction de la force détectée.

7. Système médical selon la revendication 5 ou 6,
selon lequel le mécanisme d'entraînement (10) est conçu de telle sorte qu'il modifie, tout au moins en partie, la grandeur cinématique en tant que fonction de la force détectée par le mécanisme de détection de la force (6').

8. Système médical selon l'une des revendications 5 à 7, selon lequel le mécanisme d'entraînement (10) couplé avec le dispositif médical (2) réalise un ajustement du positionnement si aucune force agissant sur le mécanisme de détection de la force (6') n'est détectée et si aucun mouvement du dispositif médical (2) n'est détecté.

9. Système médical selon l'une des revendications 5 à 8, selon lequel le mécanisme de guidage (6) est disposé au niveau du dispositif médical (2).
